# EUROPÄISCHE PATENTSCHRIFT

(11) **EP 2 991 695 B1**
(45) Veröffentlichungstag und Bekanntmachung des Hinweises auf die Patenterteilung: **20.12.2017**
(21) Anmeldenummer: 14724015.4
(22) Anmeldetag: 28.04.2014
(51) Int. Cl.: A61L 27/56, A61F 2/28, A61F 2/44

(54) **KERAMISCHES KNOCHENERSATZ-MATERIAL UND VERFAHREN ZU SEINER HERSTELLUNG**
CERAMIC BONE SUBSTITUTE MATERIAL AND METHOD FOR THE PRODUCTION THEREOF
MATÉRIAU CÉRAMIQUE DE REMPLACEMENT OSSEUX ET SON PROCÉDÉ DE FABRICATION

(30) Priorität: 30.04.2013 DE 102013007401
(43) Veröffentlichungstag der Anmeldung: 09.03.2016
(73) Patentinhaber: CeramTec GmbH, 73207 Plochingen (DE); Friedrich-Alexander-Universität Erlangen-Nürnberg, 91054 Erlangen (DE)
(72) Erfinder: WECKER, Heinrich, 90542 Eckental (DE); KELNBERGER, Alfons, 90552 Röthenbach (DE); GREIL, Peter, 91085 Weisendorf (DE); FEY, Tobias, 91058 Erlangen (DE); SCHMIDT, Johanna, 91245 Simmelsdorf (DE); ZIERATH, Bodo, 91058 Erlangen (DE)
(74) Vertreter: Uppena, Franz
(86) Internationale Anmeldenummer: PCT/EP2014/058583
(87) Internationale Veröffentlichungsnummer: WO 2014/177509

(56) Entgegenhaltungen:
- EP-A1- 1 329 229
- DE-A1- 19 753 249
- DE-A1-102008 023 911
- US-A1- 2005 228 498
- US-A1- 2011 022 180
- DAVID D. BROWN ET AL: "Investigation of Strut Crack Formation in Open Cell Alumina Ceramics", JOURNAL OF THE AMERICAN CERAMIC SOCIETY, Bd. 77, Nr. 6, Juni 1994 (1994-06), Seiten 1467-1472, XP055132041, ISSN: 0002-7820, DOI: 10.1111/j.1151-2916.1994.tb09744.x

## Beschreibung

Die Erfindung betrifft ein Verfahren zur Herstellung eines keramischen Knochenersatz-Materials sowie ein keramisches Knochenersatz-Material. Insbesondere betrifft die Erfindung die Herstellung von Knochenersatz-Material mittels Direktabformung, das als Bandscheiben-Ersatz Verwendung finden kann.

Endoprothetische Bauteile zur Fusionierung von Wirbelkörpern sind bekannt. Sie sind in ihrer Geometrie der Anatomie des menschlichen Wirbelkörpers angepasst, befinden sich zwischen zwei Wirbelköpern und ersetzen die Bandscheibe ganz oder teilweise.

Typischerweise halten sie in einer ersten Phase des Verbleibs im menschlichen Körper allein durch ihre mechanischen Eigenschaften (Tragfähigkeit) die Wirbelkörper auf Distanz und in einer anatomisch korrekten und neurologisch optimalen Position. In der Ausführungsform als Cage fördern sie die Fusionierung eines im bzw. am Implantat angelagerten Knochens und somit das Verwachsen der beiden sie umgebenden Wirbelkörper in einer zweiten Phase.

Diese bekannten Bauteile zur Fusionierung von Wirbelkörpern basieren auf metallischen Werkstoffen wie z.B. Titan oder Tantal, Kunststoffen wie PEEK oder Keramiken wie z. B. Siliziumnitrid.

Metallische Werkstoffe haben beispielsweise folgende Nachteile:
- metallischer Abrieb und daraus resultierende negative Auswirkungen auf den menschlichen Organismus, z.B. Fremdkörperreaktionen wie entzündliche oder immunologische Reaktionen
- Artefakte in der Bildgebung bei der medizinischen Diagnostik
- Alterungseffekte und Langzeitverhalten (Ermüdung, Korrosion, Freisetzung von metallischen Ionen, die toxisch wirken können)

Bauteile basierend auf Kunststoffen wie z.B. hochvernetzte PE-Werkstoffe oder PEEK können folgende Nachteile aufweisen:
- Unzureichende mechanische Eigenschaften wie z.B. das Abbrechen von Zacken oder sonstigen Bestandteilen des Bauteils beispielsweise beim Einbau. Dies kann negative Auswirkungen auf den menschlichen Organismus haben.
- Mangelnde Darstellbarkeit bei den gängigen Bildgebungsverfahren (MRI, Röntgen). Dadurch bedingt Einsatz von metallischen Markern.
- Alterungseffekte und Langzeitverhalten, insbesondere Ermüdung des Materials.

Ein grundlegendes Problem, das bei Implantations-Operationen zunehmend in den Fokus rückt, ist das Infektionsrisiko während der Operation. Dieses Risiko kann mit keramischen Bauteilen reduziert werden, deren Oberflächeneigenschaften beispielsweise hemmend auf die Besiedelung mit Bakterien wirken können.

Bekannt sind auch keramische Bauteile, beispielsweise basierend auf Siliziumnitrid.

Diese Werkstoffklasse wurde jedoch mit Blick auf exzellente Hochtemperatureigenschaften entwickelt - beispielsweise zur mechanischen Bearbeitung von metallischen Bauteilen für die Automobilindustrie - und rangiert bei den für die Anwendung als medizinisches Implantat geforderten Eigenschaften wie Festigkeit, Härte und Langzeitstabilität im Vergleich zu anderen keramischen Hochleistungswerkstoffen basierend auf oxidischen Systemen eher im Mittelfeld.

Außerdem handelt es sich um einen aus mehreren Komponenten zusammengesetzten Werkstoff mit nadelförmigen Siliziumnitridpartikeln, eingebettet in eine Glasmatrix. Entsprechend aufwändig ist die Sinterung des Werkstoffs. Ebenfalls dadurch bedingt ist die mechanische Bearbeitung - Schleifen, Polieren - extrem anspruchsvoll und schwierig.

Alle diese Nachteile erhöhen die Kosten bei der Herstellung der Bauteile, was einen weiteren Nachteil darstellt.

Darüber hinaus weisen aus Si₃N₄ gefertigte Bauteile eine eher dunkle Färbung - grau bis schwarz - auf, was aus rein optischen und ästhetischen Gründen im medizinischen Bereich auf eine geringe Akzeptanz stößt.

Bekannte keramische Cages sind in der Regel im Wesentlichen ringförmig ausgeführt bzw. der Form und Anatomie der menschlichen Wirbelkörper angepasst, wobei der Ring aus einer monolithischen, also dichten, festen und steifen Keramik besteht.

Im Zentrum können diese Cages einen Hohlraum aufweisen, der entweder mit bekannten Knochen(ersatz)-Materialien (autolog, allogen bzw. synthetisch) aufgefüllt wird oder eine künstliche poröse osseoinduktive oder osseokonduktive Kernstruktur aufweist, die in der Regel wesentlich weniger steif ist als der äußere Ring. In diesem Bereich sollen Knochenzellen neues Knochenmaterial aufbauen, wobei die daran beteiligten Zellen einen entsprechenden mechanischen Stimulus benötigen.

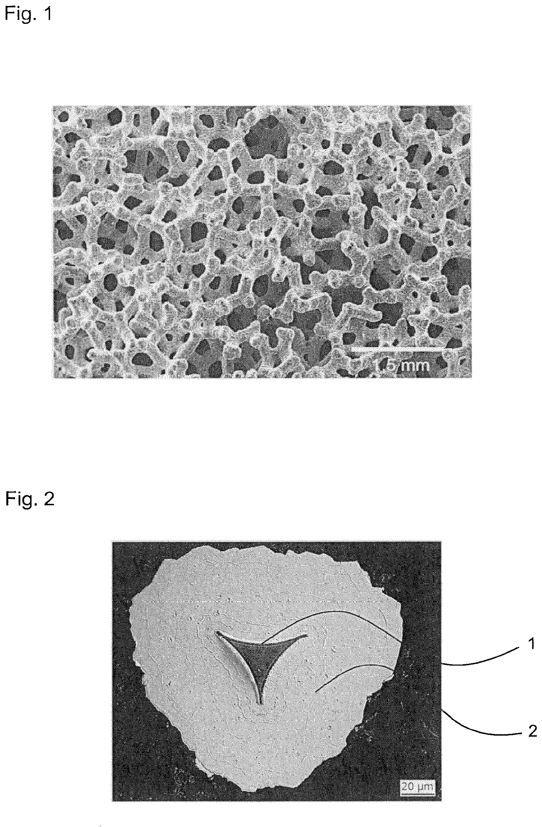

Bezüglich dieser Kernstrukturen gibt es verschiedene bekannte Herstellungsansätze:
Eine direkte Abformtechnik auf Basis von Polyurethan-Schäumen in Kombination mit einem speziellen CVD-(Chemical Vapor Deposition) Verfahren zur Abscheidung von Tantal, ist bekannt, beispielsweise aus der US 5,282,861. Mit dem Verfahren sind poröse und interkonnektierende Tantal-Strukturen herstellbar, vgl. Fig. 1, die eine Knochenneubildung unterstützen sollen. Der Herstellungs-Prozess ist sehr aufwändig, schwer kontrollierbar und nicht zuletzt aufgrund des verwendeten Materials Tantal auch teuer.

Wesentlich ist, dass interkonnektierende Strukturen, d.h. offenzellige Strukturen gebildet werden, was zum osseokonduktiven und osseoinduktiven Charakter der damit gefertigten Strukturen beiträgt.

Aufgrund des Herstellungsverfahrens sind die einzelnen Stege, welche die porenartigen Hohlräume bilden, folgendermaßen aufgebaut:
Im Zentrum befindet sich eine kohlenstoffhaltige Struktur, die aus dem Polyurethanschaum durch Pyrolyseprozesse entsteht, die im Schnitt durch den Steg eine triangelförmige Gestalt aufweist, vgl. Bezugszeichen 1 in Fig. 2.

Auf diese Strukturen wird mit einem-CVD Verfahren Tantal abgeschieden, das so eine Ummantelung 2 bildet.

Die Herstellung von osseokonduktiven Strukturen aus keramischen Werkstoffen ist ebenfalls bekannt. Eine Möglichkeit der Herstellung besteht in einem Aufschäumverfahren, bei dem durch speziell geführte Prozesse Luft in einen keramischen Schlicker eingebracht wird und somit Blasen erzeugt werden. Diese Strukturen sind mechanisch relativ stabil und belastbar und die Druckfestigkeiten liegen im zweistelligen MegaPascal Bereich.

Nachteilig ist aber, dass die Interkonnektivität der porösen Strukturen nicht oder kaum vorhanden ist, und somit eine wesentliche Voraussetzung für Knochenneubildung fehlt.

Eine weitere Variante zur gezielten Porenbildung in keramischen Strukturen basiert auf der Verwendung von organischen Porenbildnern, beispielsweise organischen Kügelchen, die im Laufe des Prozesses gezielt ein- oder aufgebracht werden und dann nach dem Ausbrennen zu Porositäten führen, vgl. z.B. DE 100 15 614 B4.

Diese Technologie ist geeignet zur Gestaltung rauer Oberflächen. Sie eignet sich aber nicht für die Herstellung von Bauteilen, die von Knochensubstanz durchbaut werden sollen, weil eine entsprechende Interkonnektivität der Poren fehlt.

US2011/022180 ein Knochenersatzmaterial, das durch infiltrieren eines Polymerschaums mit einer keramischen Suspension, trocknen und sintern erhalten wird. Die Makroporen werden anschliessend ganz oder teilweise, insbesondere an der Aussenseite, mit einem Polymeren oder einen Gemisch aus einem Polymer und keramischen Material gefüllt.

EP 1 329 229 offenbart Knochenersatzmaterial aus Calciumphosphat, die nach dem Schwartzwalder-Verfahren, d.h. durch Behandlung eines offenzelligen Polymerschaums mit einer keramischen Suspension, trocknen, ausbrennen des Schaums und sintern, gewonnen werden. Durch Verwendung von kristallinem Phosphat und eine Wiederholung der Behandlung wird eine verbesserte Festigkeit des Implantats erzielt.

DE 10 2008 023911 offenbart ein Knochenimplantat, das aus einem makro-und mikroporösen Kern und einer mikroporösen Aussenschicht besteht. Das Implantat wird erhalten durch Einbrigen eines offenzelligen Schaumstoffes in eine etwas zu grosse Form, auffüllen mit einem Schlicker, trocknen , ausbrennen und sintern. Die Aufgabe der Erfindung ist daher, ein Knochenersatz-Material bereitzustellen, das aus einer bioinerten Keramik besteht, osseokonduktive Eigenschaften, d.h. unter anderem interkonnektierende Poren aufweist und das als Bandscheiben-Implantat verwendet werden kann. Dies bedeutet auch, dass das Knochenersatz-Material trotz hoher Porositäten eine ausreichende Festigkeit aufweisen soll. Weiterhin soll ein Verfahren zur Verfügung gestellt werden, mit dem solch ein Knochenersatz-Material wirtschaftlich hergestellt werden kann.

Diese Aufgabe wird durch die Merkmale der unabhängigen Verfahrens- und Produktansprüche gelöst. Ziel ist die Herstellung poröser keramischer Knochenersatz-Materialien, die insbesondere in Wirbelsäulen-Cages Verwendung finden können. Knochenersatz-Materialien aus hochfester Al₂O₃- bzw. ZTA- (zirconia thoughened alumina) Keramik können als Ersatz beispielsweise für metallische bzw. polymerbasierte Cages dienen.

Ein erfindungsgemäßes Verfahren zur Herstellung von Knochenersatz-Material, das zumindest einen porösen keramischen osseokonduktiven Teil aufweist, umfasst demgemäß zumindest die folgenden Schritte:
a) Bereitstellen eines Schaums mit einer Porendichte von 30 bis 80 ppi;
b) Herstellen einer keramischen Infiltratsuspension,die als keramisches Material Al203-basierte Keramiken oder ZTA-Keramiken enthält;
c) Infiltrieren des Schaums mit der keramischen Infiltratsuspension;
d) Entbindern des keramischen Materials und Ausbrennen des Schaums;
e) Sintern, wobei das Sinterprozess folgende Schritte umfasst:
   e.1) Vorbrennen , vorzugsweise bei Temperaturen von bis zu 1400 bis 1500°C
   e.2) Heißisostatisches Pressen (HIP) in einer Hochdruck-Inertgasatmosphäre, vorzugsweise bei einem Druck bis zu 1400 bar und einer Temperatur bis zu 1500°C , insbesondere bevorzugt in einer Argonatmosphäre.
Das erfindungsgemäße Knochenersatz-Material umfasst zumindest einen porösen keramischen osseokonduktiven Teil, der dadurch gekennzeichnet ist, dass er eine offenporige, wabenartige Zellstruktur aufweist.

Unter einer offenporigen, wabenartigen Zellstruktur wird im Rahmen dieser Erfindung eine interkonnektierende Porosität verstanden, die ein Gerüst für die Knochenneubildung bereitstellt, also osseokonduktiv wirkt. Aus dem Stand der Technik sind Strukturen bekannt, die im Wesentlichen auf stegartigen Gerüsten aus Tantal beruhen, oder Strukturen, die aus einer massiven Keramik mit vielen eingebetteten Poren basieren. Die Poren der bekannten keramischen Strukturen sind jedoch im Wesentlichen nicht interkonnektierend. Eine erfindungsgemäße wabenartige Struktur besteht dagegen aus keramischen Stegen, die die Durchgängigkeit bzw. die Durchlässigkeit der Poren für Körperflüssigkeiten und Knochenzellen nicht behindern. Die Gesamtporosität kann zwischen 50% und 90% variieren und beträgt idealerweise 65% bis 80%. Diese Struktur führt zu einer offenzellularen, d.h. durchgängig verbundenen Porenstruktur, die die Ausbildung eines mikrobiologischen Netzwerkes und insbesondere die Vaskularisierung der sich bildenden Knochensubstanz ermöglicht. Dies stellt einen großen Vorteil gegenüber geschlossenzelligen Strukturen dar, weil damit die Vitalität der gebildeten Knochensubstanz ermöglicht wird.

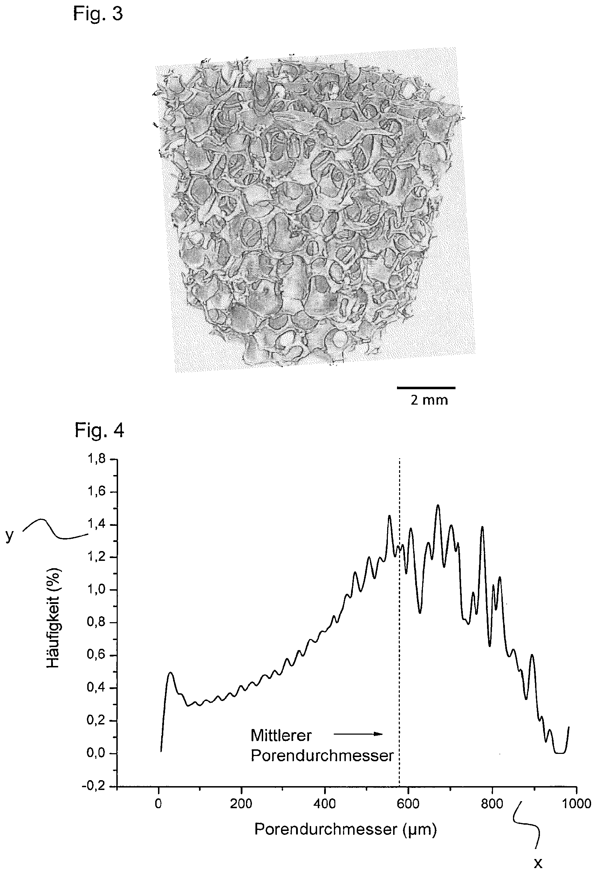

Figur 3 zeigt die offenporige, wabenförmige Struktur eines Knochenersatz-Materials, das mit einem 45 ppi (pores per inch) PU-Schaum als Templatträger hergestellt wurde in einer Röntgen-µCT-Aufnahme (Mikrofokus-Computertomographie).

Ein besonders bevorzugtes Verfahren zur Herstellung offenzellulärer Keramik-Schäume für Knochenersatz-Materialien basiert auf einem Direktabformverfahren mit einem Polyurethan-Templatträger (PU-Templatträger oder PU-Schäume), der mit einem niedrigviskosen keramischen Schlicker, auch keramische Infiltratsuspension genannt, infiltriert bzw. imprägniert wird. Als keramisches Material können vorzugsweise Al₂O₃-basierte Keramiken oder ZTA-Keramiken zum Einsatz kommen. Gemäß einer besonders bevorzugten Ausführungsform der Erfindung werden Schäume, insbesondere aus Polyurethan, als Templatträger eingesetzt. Gute Ergebnisse werden mit Schäumen einer Porendichte von 76 pores per cm (30 pores per inch (ppi)) bis 203 pores per cm (80 ppi) bevorzugt 102 bis 127 pores per cm (40 bis 50 ppi) erhalten.

Besonders vorteilhaft hat sich eine Porendichte der Schäume von 102 bis 127 pores per cm (40 bis 50 ppi) und insbesondere 114 pores per cm (45 ppi), entsprechend einer mittleren Porengröße von 600 µm, herausgestellt, weil sich damit optimale Bedingungen für die Osseointegration und Vaskularisierung einstellen lassen.

Grundsätzlich kann ein Knochenersatz-Material, das mittels der vorgenannten PU-Schäume hergestellt wurde, vorteilhaft Porengrößen in einem Bereich von 200 bis 1000 µm, bevorzugt in einem Bereich von 400 bis 600 µm und besonders bevorzugt zwischen 300 und 520 µm aufweisen. Figur 4 zeigt eine typische Porengrößenverteilung eines erfindungsgemäßen Knochenersatz-Materials, das mit einem 114 pores per cm (45 ppi) PU-Schaum nach dem erfindungsgemäßen Verfahren hergestellt wurde. Aufgetragen ist die Porengröße in µm auf der Achse x gegen die Häufigkeit in % auf der Achse y.

Gemäß einer bevorzugten Ausführungsform der Erfindung weisen die Poren eine offenzellige Polyeder-Struktur, bevorzugt eine Dodekaeder-Struktur, auf, die sich aus der Porenform der PU-Templatträger ergibt.

Zur Verbesserung der Haftfähigkeit und Aufnahme der keramischen Infiltratsuspension kann es vorteilhaft sein, eine Vorinfiltration der Schäume mit einem Primer durchzuführen. Hierzu kann beispielsweise in wässrigen Systemen ein Polyvinylalkohol (PVA) und in Systemen mit organischen Lösemitteln ein Polyvinylbutyral (PVB) verwendet werden. Organische Suspensionen ermöglichen eine höhere Feststoffbeladung bei niedriger Viskosität als wässrige Systeme. Dadurch kann eine höhere Packungsdichte in der Beschichtung erzielt werden. Durch höhere Sinterdichten der Stege können bessere mechanische Eigenschaften erzielt werden. Wässrige Systeme werden dagegen aus Umweltschutzgründen bevorzugt.

Ganz entscheidend ist die Aufbereitung und rheologische Einstellung der keramischen Infiltrationssuspension. Dazu werden als grundlegende Komponenten der Suspension keramisches Pulver, Stabilisatoren, Dispergatoren, Entschäumer und ggf. gängige Bindersysteme benutzt.

Gemäß einer besonders bevorzugten Weiterbildung der Erfindung umfasst der Schritt b) Herstellen der keramischen Infiltratsuspension folgende Schritte:
b.1) Vermischen der grundlegenden Komponenten, insbesondere ein Lösungsmittel, keramisches Pulver und optionale Komponenten wie Stabilisatoren, Dispergatoren, Entschäumer und/oder Binder;
b.2) Homogenisierung und Entgasung der Mischung in einem Intensivmischer.

Gemäß einer besonders bevorzugten Weiterbildung der Erfindung ist das Lösungsmittel Wasser. Prinzipiell können alle möglichen organischen und anorganischen Lösungsmittel verwendet werden.

Als optionale Komponenten eignen sich erfindungsgemäß: Eine mineralische Säure wie z.B. Salzsäure HCl kann als Stabilisator fungieren. Salzsäure kann auch als Dispergator genutzt werden, der für eine homogene Verteilung der keramischen Partikel sorgt und einer Agglomeration entgegenwirkt. Salzsäure mit einem Anteil von ca. 7 Vol.-% bezogen auf das Gesamtvolumen der Infiltratsuspension hat sich als besonders vorteilhaft herausgestellt.

Als Entschäumer kann vorzugsweise Octadecanol verwendet werden, eine Ölsäure, vorzugsweise mit einem Anteil von weniger als 1 Gew.-% bezogen auf das Gesamtgewicht der Infiltratsuspension. Der Entschäumer wird zur Vermeidung von Porenbildung bei der Herstellung der beschichteten PU-Schäume während des Tränkens bzw. der Infiltration der Schäume eingesetzt.

Darüber hinaus können gängige Binderorganiken zugesetzt werden, um die keramischen Partikel auf den PU-Templatträgern zu binden. Hierfür eignet sich beispielsweise ein System basierend auf PVA oder PVB.

Die mit diesen Komponenten und dem keramischen Pulver hergestellte keramische Infiltratsuspension wird zwischen einer und 96 Stunden in einem Taumelmischer oder 1 bis 10 Stunden in einem Intensivmischer bei Raumtemperatur homogenisiert und unter Anwendung eines Vakuums entgast. Für die Imprägnierung der PU Schäume ist die Entgasung besonders wichtig, um Blasenbildung in den dünnen Stegen und damit negative Auswirkungen auf die mechanischen Eigenschaften zu verhindern.

Die anschließende Infiltration der PU-Templatträger mit dieser keramischen Infiltratsuspension, Schritt c) des erfindungsgemäßen Verfahrens, erfolgt in mehreren

Schritten zwischen einem und 20 mal, wobei sich die Anzahl der Schritte nach der der gewünschten Beschichtungsdicke richtet.

Grundsätzlich richtet sich die Feststoffbeladung nach der Viskosität der Infiltratsuspension. Die Viskosität darf den Grenzwert für eine effektive Infiltration nicht überschreiten, d.h. die Feststoffbeladung sollte nur so hoch sein, dass dieser Grenzwert nicht überschritten wird. Als Feststoffbeladung der Infiltratsuspension hat sich ein Gehalt zwischen 5 und 50 Vol.-%, bezogen auf das Volumen der Infiltrationssuspension, als vorteilhaft herausgestellt; besonders vorteilhaft ist ein Wert von 20 bis 30 Vol.-%.

Damit können Beladungen des PU-Schaums zwischen 20 und 2000 Gew.% erreicht werden, wobei sich ein Wert von 800 bis 1200 Gew.-% bezogen auf die Masse des Templatträger-Schaums als besonders vorteilhaft herausgestellt hat. Über die Beladung kann die Stegdicke der Schäume erhöht werden, wodurch die Porosität des fertigen Knochenersatz-Materials gezielt eingestellt werden kann.

Gegebenenfalls erfolgt im Zustand des mit der Keramiksuspension imprägnierten Schaumes (Grünzustand) oder nach einer formstabilisierenden Glühung bei einer Temperatur über 800°C (Weißzustand) die zumindest teilweise Verfüllung oder Imprägnierung der Oberflächenporosität mit einer keramischen Masse, vorzugsweise einer Al₂O₃-umfassenden Masse, mit geeignetem rheologischem Verhalten zur Erzeugung einer für die Fügung geeigneten Oberfläche oder einem Fügebereich. Dieses Vorgehen ist insbesondere dann von Vorteil, wenn das Knochenersatz-Material integral mit einem weiteren Bauteil beispielsweise zu einem Cage zusammengefügt werden soll.

Nach der Formgebung erfolgt der Ausbrand des PU-Templatträgers. Hierbei ist eine vollständige und zerstörungsfreie Entbinderung nötig.

Dies erfolgt vorzugsweise bei Temperaturen ≤ 600°C und muss sehr vorsichtig geschehen, da die Ausgasung der organischen Bestandteile die fragilen Strukturen sonst zerstören würde.

Als besonders vorteilhaft hat sich hier eine ratenkontrollierte Entbinderung herausgestellt, bei der eine Entbinderungsrate (Materialaustrag pro Volumen und Zeit) < 0,1 Gew.-%/(cm³ h) nicht überschritten werden sollte. Insbesondere Entbinderungsraten von 0,005 bis 0,02 Gew.-%/(cm³ h) bzw. < 0,02 Gew.-%/cm³ h) haben sich als vorteilhaft erwiesen. Dieses Vorgehen erlaubt eine effiziente Vermeidung der Zerstörung der filigranen trabekulären, offenzelligen Strukturen durch eine unkontrollierte Ausgasung der organischen Bestandteile.

Der Zeitrahmen der Entbinderung ist vom Volumen des Schaumformkörpers abhängig und liegt für ein Körpervolumen von 1 cm³ bei 30 bis 50 Stunden, vorzugsweise zwischen 35 und 45 Stunden, wobei 1 bis 2 g Organik pro Formkörper ausgetragen werden.

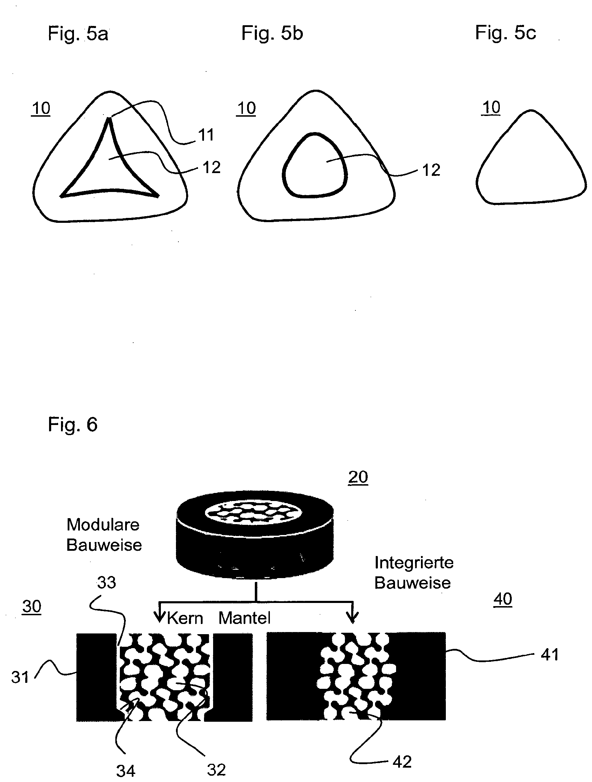

Ein ganz entscheidender Vorteil dieser Erfindung liegt auch in der speziellen thermischen Behandlung der grünen, infiltrierten Templatträger-Strukturen, die eine erhöhte mechanische Stabilität im Vergleich zu gängigen, auf Basis dieser Methode hergestellten Strukturen besitzen. Dies wird im Folgenden näher erläutert, wobei das erfindungsgemäße Vorgehen aus folgendem Dilemma resultiert:
Durch die Abformung und das anschließende Ausbrennen des PU-Schaums bilden sich in den einzelnen Stegen, die die wabenförmige oder trabekuläre Struktur aufspannen, Hohlräume - sog. Hohlstege - mit einer triangelförmigen Struktur. Die Figuren 5a und 5b zeigen diese Hohlstege 10 mit dem triangelförmigen Querschnitt.

Wie aus Fig. 5a ersichtlich, weisen die Hohlstege 10 im Querschnitt spitze Enden 11 auf. Die Hohlräume 12 als solche wirken sich generell negativ auf die mechanische Belastbarkeit der trabekulären Struktur aus. Von den spitzen Enden 11 können Risse und Materialversagen bevorzugt ausgehen.

Daraus wurde abgeleitet, dass die spitzen Enden, von denen Risse und Materialversagen ausgehen können, durch eine Anpassung der Geometrie, d.h. durch eine Verringerung der Krümmung im Kerbgrund durch Abrundung, siehe Fig. 5b, bzw. völlige Elimination der Hohlstege, siehe Fig. 5c, optimiert werden können, um die mechanische Stabilität zu erhöhen.

Die erfindungsgemäße Lösung des Problems sieht vor, die entbinderten Strukturen einem mehrstufigen Sinterprozess zu unterziehen, bei dem die Hohlstege abgerundet und zumindest teilweise eliminiert werden.

Daher wird ein mehrstufiger Sinterprozess e) verwendet, der folgende Schritte beinhaltet:
e.1) Vorbrand der Strukturen bei Temperaturen von bis zu 1400 bis 1500°C
e.2) Heißisostatisches Pressen (HIP) in einer Hochdruck-Inertgasatmosphäre, insbesondere einer Argonatmosphäre, bei einem Druck bis zu 1400 bar und einer Temperatur bis zu 1500°C über einen Zeitraum von bis zu 60 Stunden.

Durch diese erfindungsgemäße Vorgehensweise ergeben sich auf Basis eines bekannten Abformverfahrens folgende neue und erfindungsgemäße Vorteile:
Die Porosität in Hohlstegen wird auf ein Minimum reduziert und damit die Festigkeit des Knochenersatz-Materials erhöht. Gleichzeitig wird durch den mehrstufigen Sinterprozess, insbesondere durch das Hippen, die Geometrie der Hohlstege an den Spitzen so optimiert, dass eine weitere Festigkeitssteigerung erfolgt. Noch vorhandene Poren sammeln sich an den vormals spitzen Enden der Hohlstege. Somit werden genau diese spitzen Enden vermieden, die ansonsten einen Schwachpunkt in der keramischen Struktur dargestellt hätten.

Die mechanischen Festigkeiten der auf diese Weise erzeugten Strukturen lassen sich damit entscheidend erhöhen. Typische Druckfestigkeiten des Knochenersatz-Materials liegen im Bereich von 15 bis 20 MPa.

Das Knochenersatz-Material kann mit osseoinduktiven Materialien, wie beispielsweise Tricalcium-Phosphat oder Hydroxylapatit oder auch organischen osseoinduktiven Verbindungen beschichtet oder zumindest teilweise verfüllt sein.

Ein Bauteil aus dem Knochenersatz-Material kann als solches verwendet werden. Gemäß einer bevorzugten Ausführungsform der Erfindung kann das Knochenersatz-Material aber auch Teil einer Prothese, insbesondere einer Endoprothese sein.

Das Knochenersatz-Material kann demgemäß auch Teil eines Bandscheiben-Implantats sein oder als Bandscheiben-Implantat verwendet werden. Eine bevorzugte Ausführungsform eines Bandscheiben-Implantats umfasst zumindest einen lasttragenden Teil und zumindest einen porösen keramischen osseokonduktiven Teil. Der osseokonduktive Teil besteht dabei bevorzugt aus einem vorstehend beschriebenen keramischen Knochenersatz-Material. Der lasttragende Teil umfasst vorzugsweise eine dichte, im Wesentlichen nicht poröse Keramik, deren Porosität bevorzugt kleiner als 5%, besonders bevorzugt kleiner als 2% und insbesondere bevorzugt kleiner als 0,5% ist.

Der lasttragende Teil und der osseokonduktive Teil können gemäß einer Ausführungsform der Erfindung durch gemeinsames Sintern stoffschlüssig verbunden sein.

Gemäß einer weiteren Ausführungsform können der lasttragende Teil und der osseokonduktive Teil auch ein modulares System bilden, das individuell zusammengestellt werden kann. Gemäß einer besonders bevorzugten Ausführungsform kann das modulare System mittels einer Steckverbindung formschlüssig zusammengefügt werden.

Nach vorgegebener Modellgeometrie kann zunächst die Fertigung eines osseokonduktiven Teils aus Knochenersatz-Material erfolgen, das dann in einen separat hergestellten, fertig gesinterten lasttragenden Teil eingesetzt und formschlüssig mit diesem verbunden werden kann.

Dies hat den Vorteil, dass sogar während des operativen Eingriffs patientengerechte Kombinationen von Kern- und Mantelstruktur erzeugt werden können und ein hohes Maß an Flexibilität gewährleistet ist.

Eine lediglich formschlüssige Fügung, d.h. keine Verklebung, Verklemmung, Versinterung oder sonstige Verbindung, hat außerdem den Vorteil, dass die biomechanische Krafteinleitung in die Mantelstruktur (lasttragender Teil) und die Kernstruktur (osseokonduktiver Teil) des Cages voneinander entkoppelt sind, so dass die mikromechanische Stimulation des Knochenwachstums im Bereich der Kernstruktur günstig und unabhängig von der festen und sehr steifen Mantelstruktur beeinflusst werden kann.

Nach dem Sintern des osseokonduktiven Teils erfolgt gegebenenfalls eine Oberflächenbearbeitung zur Einstellung der genauen Geometrie und Toleranzen, um eine optimale Fügepassung mit dem lasttragenden Teil im modularen Aufbau zu erzielen.

Ein Absatz im Mantelbereich kann zur Lagefixierung der Kernstrukturen (osseokondutiver Teil) dienen.

Natürlich kann ein erfindungsgemäßes Bandscheiben-Implantat auch in einer integrierten Bauweise zur Verfügung gestellt werden. Hier erfolgt dann die Fertigung von lasttragendem und osseokonduktivem Teil durch separate Herstellung im Grünzustand und anschließender gemeinsamer Sinterung, die zu einer stoffschlüssigen Fügung führt.

Figur 6 zeigt beispielhaft wesentliche Unterscheidungsmerkmale von modularen und integrierten Ausführungsformen von Bandscheiben-Implantaten 20. Das Implantat 30 weist eine modulare Bauweise auf, die an dem lediglich zur Veranschaulichung deutlich sichtbaren Fügespalt 33 zwischen dem lasttragenden Teil 31 und dem osseokonduktiven Teil 32 zu erkennen ist. Im unteren Bereich des Implantats ist darüber hinaus ein Absatz 34 im lasttragenden Teil 31 zu erkennen. Die Form des osseokonduktiven Teils 32 kann an diesen Absatz 34 in verschiedener Weise angepasst werden. In Abb. 4 weist der osseokonduktive Teil 32 einen zu dem Absatz des lasttragenden Teils 34 komplementären Absatz auf. Andere, lediglich formschlüssige Alternativen sind dem Fachmann bekannt und sollen durch dieses Beispiel natürlich nicht ausgeschlossen werden.

Die integrierte Bauweise ist beispielhaft an dem Bandscheiben-Implantat 40 dargestellt. Der lasttragende Teil 41 und der osseokonduktive Teil 42 wurden im Grünzustand zusammengefügt und anschließend gemeinsam versintert. Es ergibt sich somit ein stoffschlüssiger Verbund zwischen den beiden Teilen 41 und 42.

Bei der integrierten Bauweise ist es generell wichtig, dass das Schwindungsverhalten von ossekonduktivem Teil und lasttragendem Teil in etwa ähnlich verläuft, da nur so eine Ablösung und Spaltöffnung zwischen Mantel- und Kernbereich vermieden werden können. Darüber hinaus ist nur so ein optimales Versintern der beiden Strukturen möglich.

Zur Gewährleistung einer optimalen Versinterung bzw. stoffschlüssigen Verbindung hat es sich als vorteilhaft herausgestellt, wenn der infiltrierte Schaum ca. 2 - 20 % größer ist als der dafür vorgesehene Durchmesser im grünen lasttragenden Teil. Nach dem Zusammenfügen steht der infiltrierte Schaum unter Druck und gewährleistet einen direkten Kontakt mit dem lasttragenden Teil. Damit wird der während der Sinterung notwendige Stoffaustausch mit dem lasttragenden Teil unterstützt.

## Patentansprüche

1. Verfahren zur Herstellung eines Knochenersatzmaterials, bestehend zumindest aus einem porösen keramischen osseokonduktiven Teil, umfassend zumindest die Schritte:
a) Bereitstellen eines Schaums mit einer Porendichte von 76 bis 203 pores per cm (30 bis 80 ppi);
b) Herstellen einer keramischen Infiltratsuspension, die als keramisches Material Al₂O₃-basierte Keramiken oder ZTA-Keramiken enthält;
c) Infiltrieren des Schaums mit der keramischen Infiltratsuspension;
d) Entbindern des keramischen Materials und Ausbrennen des Schaums;
e) Sintern, wobei das Sintern folgende Schritte umfasst:
e.1) Vorbrennen, vorzugsweise bei Temperaturen von bis zu 1400 bis 1500°C
e.2) Heißisostatisches Pressen in einer Hochdruck-Inertgasatmosphäre, vorzugsweise bei ≤ 1400 bar und einer Temperatur ≤ 1500°C, insbesondere bevorzugt in einer Argonatmosphäre.

2. Verfahren nach Anspruch 1, **dadurch gekennzeichnet, dass** der Schaum offenporig ist.

3. Verfahren nach Anspruch 1 oder 2, **dadurch gekennzeichnet, dass** der Schaum eine Porenflächendichte von 40 bis 50 ppi aufweist.

4. Verfahren nach einem der vorstehenden Ansprüche, **dadurch gekennzeichnet, dass** der Schritt b) Herstellen der keramischen Infiltratsuspension folgende Schritte umfasst:
b.1) Vermischen der grundlegenden Komponenten, umfassend ein Lösungsmittel, insbesondere Wasser, keramisches Pulver und optionale Komponenten wie Stabilisatoren, Dispergatoren, Entschäumer und/oder Binder;
b.2) Homogenisierung und Entgasung der Mischung in einem Taumelmischer und/oder einem Intensivmischer, vorzugsweise unter Vakuum;
b.3) Einstellen der Rheologie der Infiltratsuspension.

5. Verfahren nach einem der vorstehenden Ansprüche, **dadurch gekennzeichnet, dass** eine Feststoffbeladung der Infiltratsuspension zwischen 5 und 50 Vol.-%, bezogen auf das Volumen der Suspension, bevorzugt zwischen 20 und 30 Vol.-% liegt.

6. Verfahren nach einem der vorstehenden Ansprüche, **dadurch gekennzeichnet, dass** zumindest in Teilbereichen eine Oberflächenporosität mit einer keramischen Masse, vorzugsweise mit einer Al₂O₃-umfassenden Masse, ganz oder teilweise verfüllt wird, so dass ein Fügebereich zum Fügen mit einem weiteren Bauteil erhalten wird.

7. Verfahren nach einem der vorstehenden Ansprüche, **dadurch gekennzeichnet, dass** Verfahrensschritt d) Entbindern und Ausbrennen bei Temperaturen ≤ 600°C durchgeführt wird, wobei Entbinderungsraten von < 0,1 Gew.-%/(cm³ h), bevorzugt < 0,02 Gew.-%/(cm³ h), eingehalten werden.

8. Knochenersatz-Material, hergestellt nach dem Verfahren nach einem der Ansprüche 1 bis 7, umfassend zumindest einen porösen keramischen osseokonduktiven Teil, **dadurch gekennzeichnet, dass** der osseokonduktive Teil eine offenporige, wabenartige Zellstruktur aufweist, wobei das Knochenersatz-Material eine Druckfestigkeit von 15 bis 20 MPa aufweist.

9. Knochenersatz-Material nach Anspruch 8, **dadurch gekennzeichnet, dass** die Gesamtporosität zwischen 50% und 90% bevorzugt zwischen 65% und 80% liegt.

10. Knochenersatz-Material nach einem der Ansprüche 8 oder 9, **dadurch gekennzeichnet, dass** die Porengröße des osseokonduktiven Teils in einem Bereich von 200 bis 1000 µm, bevorzugt in einem Bereich von 400 bis 600 µm und besonders bevorzugt zwischen 300 und 520 µm liegt.

11. Bandscheiben-Implantat, umfassend zumindest einen lasttragenden Teil und zumindest einen porösen keramischen osseokonduktiven Teil, wobei der osseokonduktive Teil ein keramisches Knochenersatz-Material nach einem der Ansprüche 8 bis 10, hergestellt nach dem Verfahren nach einem der Ansprüche 1 bis 7, ist und der lasttragende Teil aus einer dichten, im Wesentlichen nicht porösen Keramik besteht, wobei die Porosität bevorzugt kleiner als 5%, besonders bevorzugt kleiner als 2% und insbesondere bevorzugt kleiner als 0,5% ist.

12. Bandscheiben-Implantat nach Anspruch 11, **dadurch gekennzeichnet, dass** der lasttragende Teil und der osseokonduktive Teil durch gemeinsames Sintern stoffschlüssig miteinander verbunden sind.

13. Bandscheiben-Implantat nach Anspruch 11, **dadurch gekennzeichnet, dass** der lasttragende Teil und der osseokonduktive Teil ein modulares System bilden, das individuell zusammengestellt werden kann und insbesondere eine formschlüssige Steckverbindung aufweisen kann.

## Claims

1. A method for producing a bone substitute material, consisting at least of one porous ceramic osseoconductive portion, comprising at least the steps:
a) provision of a foam having a pore density of 76 to 203 pores per cm (30 to 80 ppi);
b) production of a ceramic infiltrate suspension that contains as ceramic material Al₂O₃-based ceramic materials or ZTA ceramic materials;
c) infiltration of the foam with the ceramic infiltrate suspension;
d) debinding of the ceramic material and burning out of the foam;
e) sintering, wherein the sintering comprises the following steps:
e.1) prefiring, preferably at temperatures of up to 1400 to 1500°C,
e.2) hot isostatic pressing in a high-pressure inert gas atmosphere, preferably at ≤ 1400 bar and a temperature ≤ 1500°C, in particular preferably in an argon atmosphere.

2. A method according to claim 1, **characterised in that** the foam is open-pored.

3. A method according to claim 1 or 2, **characterised in that** the foam has a pore-surface density of 40 to 50 ppi.

4. A method according to one of the preceding claims, **characterised in that** step b) production of the ceramic infiltrate suspension comprises the following steps:
b.1) blending of the basic components, comprising a solvent, in particular water, ceramic powder and optional components, such as stabilizers, dispersants, defoamers and/or binders;
b.2) homogenization and degassing of the mixture in a tumbling mixer and/or an intensive mixer, preferably under vacuum;
b.3) setting of the rheology of the infiltrate suspension.

5. A method according to one of the preceding claims, **characterised in that** a solids loading of the infiltrate suspension lies between 5 and 50% by volume, relative to the volume of the suspension, preferably between 20 and 30% by volume.

6. A method according to one of the preceding claims, **characterised in that** at least in partial regions a surface porosity is completely or partly filled with a ceramic mass, preferably with a mass comprising Al₂O₃, so that a joining region for joining to another component is obtained.

7. A method according to one of the preceding claims, **characterised in that** method step d) debinding and burning out is carried out at temperatures ≤ 600°C, wherein debinding rates of < 0.1 % by weight/(cm³ h), preferably < 0.02% by weight/(cm³ h), are observed.

8. A bone substitute material produced according to the method according to one of claims 1 to 7, comprising at least one porous ceramic osseoconductive portion, **characterised in that** the osseoconductive portion has an open-pored, honeycomb-like cell structure, wherein the bone substitute material has a compressive strength of 15 to 20 MPa.

9. A bone substitute material according to claim 8, **characterised in that** the total porosity lies between 50% and 90%, preferably between 65% and 80%.

10. A bone substitute material according to one of claims 8 or 9, **characterised in that** the pore size of the osseoconductive portion lies in a range of 200 to 1000 µm, preferably in a range of 400 to 600 µm, and particularly preferably between 300 and 520 µm.

11. An intervertebral disk implant, comprising at least one load-bearing portion and at least one porous ceramic osseoconductive portion, wherein the osseoconductive portion is a ceramic bone substitute material according to one of claims 8 to 10, produced according to the method according to one of claims 1 to 7, and the load-bearing portion consists of a dense, substantially nonporous ceramic material, wherein the porosity is preferably smaller than 5%, particularly preferably smaller than 2%, and in particular preferably smaller than 0.5%.

12. An intervertebral disk implant according to claim 11, **characterised in that** the load-bearing portion and the osseoconductive portion are connected together in a substance-locking manner by means of joint sintering.

13. An intervertebral disk implant according to claim 11, **characterised in that** the load-bearing portion and the osseoconductive portion form a modular system which can be individually compiled and in particular can have a form-locking plug connection.

## Revendications

1. Procédé de fabrication d'un matériau substitut osseux, formé au moins d'une partie ostéo-conductrice en céramique poreuse, lequel procédé comporte au moins les étapes suivantes :
a) prendre une mousse qui présente une densité de pores de 76 à 203 pores par centimètre (30 à 80 pores par pouce),
b) préparer une suspension de céramique d'infiltration, qui contient, en tant que matériau céramique, une céramique à base d'alumine ou une céramique d'alumine à renfort de zircone (ZTA),
c) faire s'infiltrer dans la mousse la suspension de céramique d'infiltration,
d) opérer un déliantage du matériau céramique et une calcination de la mousse,
e) et opérer un frittage, lequel frittage comporte les étapes suivantes :
e.1) pré-calcination, de préférence à des températures allant jusqu'à 1400 à 1500 °C,
e.2) et compression isostatique à chaud sous atmosphère de gaz inerte sous haute pression, de préférence sous une pression inférieure ou égale à 1400 bars et à une température inférieure ou égale à 1500 °C, et en particulier sous atmosphère d'argon.

2. Procédé conforme à la revendication 1, **caractérisé en ce que** la mousse est une mousse à pores ouverts.

3. Procédé conforme à la revendication 1 ou 2, **caractérisé en ce que** la mousse présente une densité superficielle de pores de 40 à 50 pores par pouce.

4. Procédé conforme à l'une des revendications précédentes, **caractérisé en ce que** l'étape (b) de préparation de la suspension de céramique d'infiltration comporte les opérations suivantes :
b.1) mélange des composants de base comprenant un solvant, en particulier de l'eau, une poudre de céramique, et des composants optionnels tels que stabilisants, dispersants, agents démoussants et/ou liants,
b.2) homogénéisation et dégazage du mélange dans un mélangeur excentrique ou un mélangeur intensif, de préférence sous vide,
b.3) ajustement des caractéristiques rhéologiques de la suspension d'infiltration.

5. Procédé conforme à l'une des revendications précédentes, **caractérisé en ce que** la charge en matières solides de la suspension d'infiltration vaut entre 5 et 50 %, en volume rapporté au volume de la suspension, et de préférence entre 20 et 30 % en volume.

6. Procédé conforme à l'une des revendications précédentes, **caractérisé en ce que**, au moins dans des domaines partiels, l'ensemble ou une partie des pores superficiels sont remplis avec une masse céramique, de préférence avec une masse comprenant de l'alumine, de telle sorte que soit obtenu un domaine de jonction pour la jonction avec un autre élément constitutif.

7. Procédé conforme à l'une des revendications précédentes, **caractérisé en ce que** l'étape de procédé (d), déliantage et calcination, est effectuée à des températures inférieures ou égales à 600 °C, ce qui fait qu'on obtient des vitesses de déliantage inférieures à 0,1 % en poids par centimètre-cube et par heure, et de préférence inférieures à 0,02 % en poids par centimètre-cube et par heure.

8. Matériau substitut osseux fabriqué selon un procédé conforme à l'une des revendications 1 à 7, comprenant au moins une partie ostéo-conductrice en céramique poreuse, **caractérisé en ce que** la partie ostéo-conductrice présente une structure alvéolaire de type nid d'abeilles à pores ouverts, le matériau substitut osseux présentant une résistance à la compression de 15 à 20 MPa.

9. Matériau substitut osseux conforme à la revendication 8, **caractérisé en ce que** la porosité totale vaut entre 50 et 90 %, et de préférence entre 65 et 80 %.

10. Matériau substitut osseux conforme à la revendication 8 ou 9, **caractérisé en ce que** la taille des pores de la partie ostéo-conductrice se situe dans un intervalle allant de 200 à 1000 µm, de préférence dans un intervalle allant de 400 à 600 µm, et mieux encore entre 300 et 520 µm.

11. Prothèse de disque intervertébral, comprenant au moins une partie support de charge et au moins une partie ostéo-conductrice en céramique poreuse, laquelle partie ostéo-conductrice est en un matériau substitut osseux céramique, conforme à l'une des revendications 8 à 10 et préparé selon un procédé conforme à l'une des revendications 1 à 7, et laquelle partie support de charge est en une céramique dense qui n'est pratiquement pas poreuse, sa porosité étant de préférence inférieure à 5 %, mieux encore, inférieure à 2 %, et surtout, inférieure à 0,5 %.

12. Prothèse de disque intervertébral conforme à la revendication 11, **caractérisée en ce que** la partie support de charge et la partie ostéo-conductrice sont liées l'une à l'autre par interpénétration de matériaux en étant frittées ensemble.

13. Prothèse de disque intervertébral conforme à la revendication 11, **caractérisée en ce que** la partie support de charge et la partie ostéo-conductrice forment un système modulaire qui peut être assorti individuellement et peut, en particulier, présenter une liaison à prise par accouplement de formes.
